(19) Europäisches Patentamt
European Patent Office
Office européen des brevets

(11) Veröffentlichungsnummer: **0 471 660 B1**

(12) **EUROPÄISCHE PATENTSCHRIFT**

(45) Veröffentlichungstag der Patentschrift: **23.08.95**

(51) Int. Cl.6: **A61K 38/48**, A61K 38/43, A61K 35/16

(21) Anmeldenummer: **91890012.7**

(22) Anmeldetag: **28.01.91**

Teilanmeldung 92117248.2 eingereicht am 28/01/91.

(54) **Protein C zur Schmerzbekämpfung.**

(30) Priorität: **16.08.90 AT 1697/90**

(43) Veröffentlichungstag der Anmeldung:
**19.02.92 Patentblatt 92/08**

(45) Bekanntmachung des Hinweises auf die
Patenterteilung:
**23.08.95 Patentblatt 95/34**

(84) Benannte Vertragsstaaten:
**AT BE CH DE DK ES FR GB IT LI LU NL SE**

(56) Entgegenhaltungen:
EP-A- 0 326 014
WO-A-90/08556

CHEMICAL ABSTRACTS, Band 93, Nr. 17, 27. Oktober 1980, Columbus, Ohio, USA P.C.COMP et al. "Evidence for multiple roles for activated protein C infibrinolysis" Seite 424, rechte Spalte, Zusammenfassung-Nr. 165 257x

CHEMICAL ABSTRACTS, Band 95, Nr. 25, 21. Dezember 1981, Columbus, Ohio, USA P. C. COMP et al. Seite 347, rechte Spalte, Zusammenfassung -Nr. 218 287g

(73) Patentinhaber: **IMMUNO Aktiengesellschaft**
**Industriestrasse 67**
**A-1221 Wien (AT)**

(72) Erfinder: **Eibl, Johann, Dr.**
**Gustav Tschermakgasse 2**
**A-1180 Wien (AT)**
Erfinder: **Pichler, Ludwig, Prof. Dr.**
**Gusshausstrasse 24**
**A-1040 Wien (AT)**
Erfinder: **Schwarz, Hans-Peter, Doz. Dr.**
**Schindlergasse 32**
**A-1180 Wien (AT)**

(74) Vertreter: **Wolfram, Gustav, Dipl.-Ing.**
**Patentanwälte**
**Sonn, Pawloy, Weinzinger & Wolfram,**
**Riemergasse 14**
**A-1010 Wien (AT)**

**Beschreibung**

Die Erfindung betrifft die Verwendung von Protein C zur Herstellung eines Arzneimittels mit antinociceptiver Wirkung.

Protein C ist ein Vitamin-K-abhängiges Glykoprotein, das in der Leber synthetisiert wird und im Plasma in einer Konzentration von 4 μg/ml als inaktives Zymogen zirkuliert. Es wird an der Gefäßwandoberfläche (Endothel) durch den Thrombin-Thrombomodulinkomplex in die aktive Serinprotease (aktiviertes Protein C) übergeführt. Es ist bekannt, daß aktiviertes Protein C profibrinolytische Eigenschaften hat. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Die Aktivierung von Protein C in vivo stellt eine negative Feedback-Reaktion der Thrombingenerierung dar. Um optimale biologische Aktivität zu entfalten, ist ein Kofaktor (Protein S) notwendig.

In der österreichischen Patentanmeldung A 1551/89 (veröffentlicht z.B. als EP-A-406 216) ist eine pharmazeutische Präparation beschrieben, die Protein C enthält und zur Behandlung bzw. Verhinderung von Thrombosen und thromboembolischen Komplikationen eingesetzt werden kann.

Da sowohl das Protein C als auch das aktivierte Protein C als körpereigene Proteine anzusehen sind, besitzen pharmazeutische Präparationen auf Basis dieser Proteine den großen Vorteil, daß sie praktisch ohne Nebenwirkungen verabreicht werden können.

Die Erfindung stellt sich die Aufgabe, das therapeutische Anwendungsgebiet von Protein C bzw. von aktiviertem Protein C zu vergrößern und sie besteht darin, daß Protein C oder aktiviertes Protein C zur Herstellung einer pharmazeutischen Präparation mit antinociceptiver Wirkung verwendet wird.

Die Erfindung beruht auf der Erkenntnis, daß die durch entzündliche Prozesse induzierte, erhöhte Schmerzempfindung mittels Protein C oder mittels aktiviertem Protein C herabgesetzt werden kann. Sie umfaßt somit auch die Verwendung von Protein C oder aktiviertem Protein C zur Herstellung einer Präparation zur Behandlung schmerzhafter Zustände, die durch akute oder chronische entzündliche Prozesse hervorgerufen werden, wobei die schmerzhaften Zustände durch post-traumatische, post-operative oder durch primäre oder sekundäre maligne Erkrankungen ausgelöst werden können.

Protein C oder aktiviertes Protein C kann insbesondere zur Herstellung einer Präparation verwendet werden, die sich zur Behandlung jener schmerzhaften Zustände eignet, welche durch rheumatoide Arthritis, Myositis, Gastritis, Kolitis oder durch Entzündungen im Urogenitaltrakt ausgelöst werden, oder im Rahmen akuter oder chronischer Transplantatabstoßung auftreten.

Es hat sich weiters gezeigt, daß Protein C oder aktiviertes Protein C imstande ist, die Adhäsion von Leukozyten an den Gefäßwänden zu verhindern und das Wandern der Leukozyten durch die Gefäßwände zu hemmen. Beide Proteine unterbinden die Aktivierung der Leukozyten und verhindern die Komplementaktivierung.

Die Erfindung betrifft auch die Verwendung von Protein C oder aktiviertem Protein C zur Herstellung eines pharmazeutischen Kombinationspräparates mit mindestens einem Analgetikum. Es hat sich gezeigt, daß ein derartiges Kombinationspräparat synergistische Effekte aufweist.

Die Gewinnung von Protein C und von aktiviertem Protein C, sowie die Prüfung auf ihre antinociceptive Wirkung werden nachfolgend beschrieben.

Gewinnung von Protein C

Hochreines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 μg menschlichem Protein C in zweiwöchigen Abständen durch intraperitoneale Injektion immunisiert. Nach sechs Wochen wurden noch einmal 50 μg des menschlichen Protein C injiziert und drei Tage später die Fusion vorgenommen. Die Myelomzellinie (P3-X-63-AG8-653, $1,5 \times 10^7$ Zellen) wurde vermischt mit $1,7 \times 10^8$ Milzzellen von einer Maus, und die Fusion nach modifizierter Köhler & Milstein-Methode unter Verwendung von PEG 1500 durchgeführt (Köhler G., Milstein C., Nature 256-(1975)495-497).

Positive Klone, getestet mittels eines ELISA, wurden zweimal subgeklont. Aszitesproduktion erfolgte durch Injektion von $5 \times 10^6$ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauffolgend Chromatographie an Sephadex G200 gereinigt. Um das Risiko der Übertragung muriner Viren zu reduzieren, wurde der Antikörper vor der Immobilisierung noch einem Virusinaktivierungsschritt unterworfen. Die so erhaltenen monoklonalen Antikörper gegen Protein C wurden an CNBR-Sepharose 4B (Pharmacia) gekoppelt. Für die Reinigung des Protein C mittels Affinitätschromatographie wurden folgende Puffer verwendet:

Als Absorptionspuffer: 20 mmol Tris, 2 mmol EDTA, 0,25 mol NaCl und 5 mmol Banzamidin;

als Waschpuffer wurde verwendet: 20 mmol Tris, 1 mol NaCl, 2 mmol Benzamidin, 2 mmol EDTA; der pH-Wert betrug 7,4;

als Elutionspuffer wurde verwendet: 3 mol NaSCN, 20 mmol Tris, 1 mol NaCl, 0,5 mmol Benzamidin, 2 mmol EDTA.

Im einzelnen: Das Prothrombinkomplex-Konzentrat wurde im Absorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein Konzentrat filtriert, bei 20.000 rpm 15 min lang zentrifugiert und durch ein 0,8 $\mu$m Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 mol/l Tris, 0,15 mol Glycin und 1 mmol EDTA, pH 8,3) dialysiert. Der Protein C-Gehalt wurde antigenmäßig mittels der Methode nach Laurell und aktivitätsmäßig nach Protac-Aktivierung bestimmt.

Das erhaltene Protein C-Eluat wurde in folgender Weise zu einer pharmazeutisch applizierbaren Präparation fertiggestellt:

Das Eluat wurde zuerst einem Ultrafiltrations- und einem Diafiltrationsschritt unterworfen. Für die Diafiltration wurde ein Puffer mit einem pH-Wert von 7,4 verwendet, der pro Liter 150 mMol NaCl und 15 mMol Trinatriumcitrat.2H$_2$O enthielt. Das erhaltene Filtrat wurde gefriergetrocknet und durch eine einstündige Dampfbehandlung bei 80° C ± 5° C und 1375 ± 35 mbar virusinaktiviert.

Das lyophilisierte virusinaktivierte Material wurde sodann in einer sterilen isotonen NaCl-Lösung gelöst und mittels Ionenaustauschchromatographie an Q-Sepharose wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mMol NaCl und 15 mMol Trinatriumcitrat zugegeben. Der pH-Wert der Lösung betrug 7,5. Maus-Immunglobulin sowie die Faktoren II, VII, IX und X konnten nicht nachgewiesen werden. Anschließend wurde die Lösung sterilfiltriert, abgefüllt und lyophilisiert. Die spezifische Aktivität betrug 14 Einheiten Protein C/mg. Eine Einheit Protein C Aktivität ist definiert als die Protein-C-Aktivität in 1 ml Normalplasma und wird gegen den ersten internationalen Standard von Protein C kalibriert. Als Aktivitätstest wurde ein amidolytischer Test verwendet, wobei das Protein C mittels Protac (Fa. Pentapharm) aktiviert wurde.

Gewinnung von aktiviertem Protein C

Die Aktivierung des gereinigten Protein C erfolgte dadurch, daß 70 ml Thrombin (500 NIH Einheiten/ml, etwa 2000 NIH Einheiten/mg Protein entsprechend) an CNBR Sepharose 4B (Pharmacia) gekoppelt wurden, worauf Protein C mit dem Thrombingel in einem Verhältnis von etwa 6 Einheiten Protein C zu 1 Einheit Thrombin bei 37° C gemischt und unter kontinuierlichem Schütteln 3 h in Reaktion belassen wurden. Die Protein C-Aktivität wurde anschließend mit chromogenem Substrat (S 2366) bestimmt. Das aktivierte Protein C wurde anschließend sterilfiltriert und durch Abfüllung und Lyophilisation zu einer pharmazeutischen Präparation fertiggestellt.

Antinociceptive Wirkung

Die antinociceptive Wirkung wurde an der entzündeten Rattenpfote durch Bestimmung der Schmerzschwelle, mit und ohne Gabe von Protein C/aktiviertem Protein C, nachgewiesen.

Die Schmerzschwelle wurde analog der von Randall und Selitto beschriebenen Methode gemessen (Randall LO and Selitto JJ: A method for measurement of analgesic activity on inflamed tissue, Arch. Int. Pharmacodyn. 111, 409-419, 1957). Dazu wurde ein Quecksilbermanometer mit einer 10 ml Spritze verbunden, deren Kolben mit einem kurzen, geschoßförmigen Zapfen ausgerüstet war. Durch diese Spitze wurde steigender Druck auf die Rattenpfote ausgeübt (20 mmHg/Sekunde). Die Schmerzschwelle wurde angegeben als jener Druck in mmHg, der notwendig ist, um die Fluchtreaktion der Pfote auszulösen.

Zur Untersuchung wurden weibliche Sprague-Dawley Ratten mit einem Gewicht zwischen 250 und 350 g verwendet. Die Entzündung wurde mittels 3 mg Carrageenan (Sigma, C-1013), aufgelöst in 100 $\mu$l

EP 0 471 660 B1

isotoner Kochsalzlösung, durch intraplantare Injektion in die Rattenpfote ausgelöst.

Die Schmerzschwelle wurde vor der Injektion (Kontrollwert) und stündlich bis zu 6 Stunden nach der Injektion von Carrageenan gemessen. Die Schmerzschwelle, die nach der Carrageenaninjektion gemessen wurde, wurde ausgdrückt in Prozent des Kontrollwertes.

Protein C oder aktiviertes Protein C wurden intravenös in eine Schwanzvene unmittelbar nach der Carrageenaninjektion injiziert. Das Injektionsvolumen betrug 2 ml/kg.

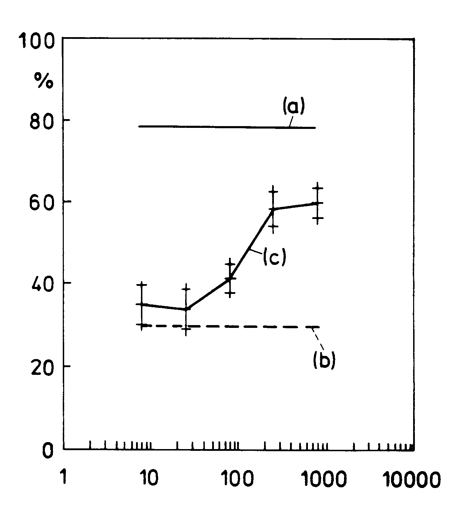

Die Ergebnisse sind in der Figur als Diagramm dargestellt, wobei als Ordinate die Schmerzschwelle in Prozent des Kontrollwertes und als Abszisse die Dosis von Protein C (Einheiten per kg) logarithmisch angegeben werden. Die Linien (a) und (b) entsprechend den Schmerzschwellen, die ohne bzw. mit Carrageenan-Einfluß gemessen wurden. Die Kurve (c) zeigt, daß die unter Carrageenan-Einfluß reduzierte Schmerzschwelle durch Gabe von Protein C angehoben wird (antinociceptiver Effekt). Die Kurve (c) gibt weiters den Streubereich der antinociceptiven Wirkung an, die durch Protein C-Dosen zu 8 E/kg (10 Tiere), zu 25 E/kg (15 Tiere), zu 80 E/kg (20 Tiere), zu 250 E/kg (20 Tiere) und zu 800 E/kg (15 Tiere) erhalten wurde. Der antinociceptive Effekt von Protein C ist somit dosisabhängig und signifikant ($p < 0,01$ für 80 Einheiten/kg; $p < 0,001$ für 250 und 800 Einheiten/kg).

Auch subkutan gegeben läßt sich dieser Effekt für Protein C nachweisen.

Es konnte weiters nachgewiesen werden, daß auch aktiviertes Protein C in einer Dosis von 250 E/kg 2 Stunden nach der intraplantaren Injektion von Carrageenan eine antinociceptive Wirkung besitzt. Aktiviertes Protein C in einer Dosis von 250 E/kg intravenös 10 Minuten vor der Carrageenan-ausgelösten Entzündung hat ebenfalls einen antinociceptiven Effekt, der 2 Stunden anhält. Der antinociceptive Effekt von aktiviertem Protein C ist ebenfalls dosisabhängig.

Histologische Untersuchungen der Rattenpfoten, die mit Protein C oder aktiviertem Protein C behandelt wurden, zeigten eine verringerte perivaskuläre Leukozytenzahl im Vergleich zu unbehandelten Tieren.

Es hat sich weiters gezeigt, daß der nicht selektive $\beta$-Adrenozeptorenblocker Propranolol die antinociceptive Wirkung von Protein C (800 E/kg) aufhebt. Die Protein C-antagonisierende Wirkung von Propranolol konnte im Dosisbereich von 0,03 bis 1 mg/kg i.v. nachgewiesen werden (siehe Tabelle 1).

Tabelle 1

| Propranolol (Ergebnisse in % der Schmerzschwelle, gemessen 3 h nach Carrageenan im Rattenpfotenmodell wie oben beschrieben) | | |
|---|---|---|
| | | Anzahl |
| Carrageenan allein | 21 ± 3 % | 15 |
| Carrageenan + Protein C (800 E/kg) | 60 ± 6 % | 15 |
| Carrageenan + Protein C + Propranolol (1 mg/kg) | 28 ± 5 % | 10 |

Dieser Antagonismus ist aber nicht auf Propranolol beschränkt, sondern auch andere $\beta$-blockierende Substanzen antagonisieren dosisabhängig die Protein C-Wirkung, wie in Tabelle 2 für Pindolol gezeigt.

Tabelle 2

| Pindolol (Ergebnisse in % der Schmerzschwelle, gemessen 3 h nach Carrageenan im Rattenpfotenmodell wie oben beschrieben) | | |
|---|---|---|
| | | Anzahl |
| Carrageenan allein | 22 ± 1,3 % | 10 |
| Carrageenan + Protein C (800 E/kg) | 65 ± 2,0 % | 10 |
| Carrageenan + Protein c + Pindolol (0,3 mg/kg) | 24 ± 2,0 % | 5 |

Nach Ausschaltung des sympathischen Nervensystems durch chemische Sympathektomie mit Reserpin (7,5 mg/kg i.p., 18 bis 24 h) und alpha-Methyl-p-tyrosin (250 mg/kg i.p., 5 h) ist Protein C stark antinociceptiv wirksam. Aber auch an diesen sympathektomierten Tieren antagonisiert Pindolol den Effekt von Protein C völlig (Tabelle 3). Somit kann eine Beteiligung des präsynaptischen Sympathikus an der

4

Wirkung von Protein C ausgeschlossen werden. Der Effekt von Protein C ist daher durch direkte Wirkung an postsynaptischen $\beta$-Adrenorezeptoren bedingt.

## Tabelle 3

## Reserpin und alpha-Methyl-p-tyrosin (Ergebnisse in % der Schmerzschwelle, gemessen 3 h nach Carrageenan im Rattenpfotenmodell wie oben beschrieben)

Ausgangswerte (20) bei: 39 ± 1,2 mmHg

|  |  | Anzahl |
|---|---|---|
| NaCl Kontr. | 61,0 ± 6,8 % | 5 |
| Carrageenan allein | 34,0 ± 10,3 % | 5 |
| Carrageenan + Protein C (800 E/kg) | 106,7 ± 18,1 % | 5 |
| Carrageenan + Protein C + Pindolol | 33,3 ± 4,6 % | 5 |
|  | (1 mg/kg i.v.) |  |

Der Effekt von Protein C ist durch $\beta$-Sympathomimetika nachzuahmen. Fenoterol, entweder mit 5 mg/kg zu Versuchsbeginn oder mit 3 mg/kg zum Zeitpunkt "3 h" i.v. injiziert, führt zu deutlicher Antinonzizeption. Somit führt die Stimulierung postsynaptischer $\beta$-Adrenozeptoren zu antinociceptiver Wirkung.

Die biochemische Wirkung des $\beta$-sympathomimetischen Effektes von Protein C wird durch Bindung an adrenergen $\beta$-Rezeptoren und Stimulierung des membrangebundenen Enzyms Adenylcyclase, welches die intrazelluläre Bildung von cAMP aus ATP katalysiert, hervorgerufen. cAMP aktiviert Protein-phosphokinasen, die ihrerseits durch Phosphorylierung inaktive in aktive Enzyme überführen. Auf diese Weise werden z.B. Lipolyse und Glukogenolyse $\beta$-sympathomimetisch gesteigert.

Die physiologischen Auswirkungen von Protein C aufgrund seiner ß-sympathomimetischen Wirkung sind vielfältig. Die Erhöhung von cAMP in den Thrombozyten führt zu einer Hemmung der Thrombozytenfunktion und zu einer Hemmung von Thrombinbindung an die Thrombozytenoberfläche. Somit kann Protein C zum Einsatz kommen, um thrombozytenabhängige Blutgerinnselbildungen, besonders im arteriellen Gefäßsystem durch Bindung an die thrombozytären ß-Adrenozeptoren zu verhindern. Die ß-adrenozeptor-positive Wirkung von Protein C hat am Herzen einen positiv inotropen Effekt und wirkt auf die glatte Muskulatur (z.B. an den Bronchiolen) erschlaffend. Aufgrund der ß-sympathomimetischen Wirkung am Gefäßsystem kann Protein C therapeutisch bei peripheren Durchblutungsstörungen eingesetzt werden. Es hat sich auch gezeigt, daß die intrakoronare Gabe von aktiviertem Protein C bei Hunden zu einer Steigerung des koronaren Blutflusses aufgrund der vasodilatatorischen Wirkung führt. Die vasodilatatorische Wirkung von Protein C kann zur Behandlung der Hypertonie herangezogen werden. Der ß-sympathomimetischen Wirkung von Protein C kann auch eine entzündungshemmende Eigenschaft zugeschrieben werden. Die ß-sympathomimetische Wirkung von Protein C führt zu einer Hemmung der Histaminfreisetzung in der Lunge. Durch die ß-sympathomimetische Wirkung von Protein C kann Protein C als Tokolytikum Anwendung finden.

**Patentansprüche**

1. Verwendung von Protein C oder aktiviertem Protein C zur Herstellung einer pharmazeutischen Präparation mit antinociceptiver Wirkung.

2. Verwendung von Protein C oder aktiviertem Protein C nach Anspruch 1 zur Herstellung einer Präparation zur Behandlung schmerzhafter Zustände, die durch akute oder chronische entzündliche Prozesse hervorgerufen werden.

3. Verwendung von Protein C oder aktiviertem Protein C nach Anspruch 2, wobei die schmerzhaften Zustände durch post-traumatische, post-operative oder durch primäre oder sekundäre maligne Erkrankungen ausgelöst werden.

4. Verwendung von Protein C oder aktiviertem Protein C nach Anspruch 2, wobei die schmerzhaften Zustände durch rheumatoide Arthritis, Myositis, Gastritis, Kolitis oder durch Entzündungen im Urogenitaltrakt ausgelöst werden.

5. Verwendung von Protein C oder aktiviertem Protein C nach Anspruch 2, wobei die entzündlichen Prozesse im Rahmen akuter oder chronischer Transplantatabstoßung auftreten.

6. Verwendung von Protein C oder aktiviertem Protein C zur Herstellung eines pharmazeutischen Kombinationspräparates mit mindestens einem Analgetikum.

**Claims**

1. The use of protein C or activated protein C for preparing a pharmaceutical preparation having an antinociceptive effect.

2. The use of protein C or activated protein C according to claim 1 for preparing a preparation destined to treat pain caused by acute or chronic inflammatory processes.

3. The use of protein C or activated protein C according to claim 2, wherein said pain is caused by post-traumatic, post-operative conditions or by primary or secondary malignant diseases.

4. The use of protein C or activated protein C according to claim 2, wherein the pain is caused by rheumatoid arthritis, myositis, gastritis, colitis or inflammations in the urogenital tract.

5. The use of protein C or activated protein C according to claim 2, wherein said inflammatory processes are associated with acute or chronic graft rejections.

6. The use of protein C or activated protein C for preparing a combined pharmaceutical preparation including at least one analgesic.

**Revendications**

1. Utilisation de la protéine C ou de la protéine C activée pour la production d'une préparation pharmaceutique à effet antinociceptif.

2. Utilisation de la protéine C ou de la protéine C activée selon la revendication 1 pour la production d'une préparation pour le traitement des états douloureux qui sont provoqués par des processus inflammatoires aigus ou chroniques.

3. Utilisation de la protéine C ou de la protéine C activée selon la revendication 2, dans laquelle les états douloureux sont déclenchés par des maladies post-traumatiques, postopératoires ou par des maladies malignes primaires ou secondaires.

4. Utilisation de la protéine C ou de la protéine C activée selon la revendication 2, dans laquelle les états douloureux sont provoqués par la polyarthrite rhumatoïde, la myosite, la gastrite, la colite ou par des inflammations des voies urogénitales.

5. Utilisation de la protéine C ou de la protéine C activée selon la revendication 2, dans laquelle les processus inflammatoires apparaissent dans le cadre d'un rejet de greffe aigu ou chronique.

6. Utilisation de la protéine C ou de la protéine C activée pour la production d'une préparation pharmaceutique combinée contenant au moins un analgésique.